# EUROPEAN PATENT SPECIFICATION

(11) **EP 4 360 568 B1**
(45) Date of publication and mention of the grant of the patent: **29.10.2025**
(21) Application number: 22841147.6
(22) Date of filing: 22.06.2022
(51) Int. Cl.: A61B 17/00, A61B 34/30

(54) **TRANSMISSION CONNECTION STRUCTURE OF INSTRUMENT DRIVER AND STERILE ADAPTER, AND SURGICAL ROBOT**
ÜBERTRAGUNGSVERBINDUNGSSTRUKTUR EINES INSTRUMENTENTREIBERS UND STERILADAPTER SOWIE CHIRURGISCHER ROBOTER
STRUCTURE DE CONNEXION DE TRANSMISSION D'UN PILOTE D'INSTRUMENT ET D'UN ADAPTATEUR STÉRILE, ET ROBOT CHIRURGICAL

(30) Priority: 14.07.2021 CN 202110795340
(43) Date of publication of application: 01.05.2024
(73) Proprietor: Cornerstone Technology (Shenzhen) Limited, Shenzhen, Guandong 518066 (CN)
(72) Inventor: YANG, Qiusheng, Shenzhen, Guangdong 518066 (CN)
(74) Representative: Dennemeyer & Associates S.A.
(86) International application number: PCT/CN2022/100523
(87) International publication number: WO 2023/284507

(56) References cited:
- WO-A1-2020/069430
- WO-A1-2021/037170
- WO-A1-2021/137107
- WO-A2-2011/037394
- WO-A2-2019/108437
- CN-A- 104 783 844
- CN-A- 105 611 894
- CN-A- 108 348 133
- CN-A- 112 957 127
- CN-A- 113 349 935
- CN-A- 113 349 937
- CN-A- 113 367 797
- CN-A- 113 367 798
- CN-U- 216 317 968
- US-A1- 2010 175 701
- US-A1- 2021 093 398

## Description

### TECHNICAL FIELD

The present application relates to the field of medical device technology, specifically to a transmission and connection structure, and a surgical robot including the same.

### BACKGROUND

A surgical robot can help a doctor achieve precise positioning in surgeries, with the advantages of reducing patient wounds and shortening postoperative recovery time. And the surgical robot has a stable operating platform that can solve problems caused by trembling and other situations of the doctor, so that the surgical robot is widely applied in clinical surgical operations. The surgical robot on a patient side is configured to perform surgical operations using surgical tools with at least one end effector. To meet the usage needs of different surgical instruments during surgery, surgical instruments and instrument drives are generally designed to be detachable for replacement of different surgical instruments during surgery. In addition, surgical instruments are generally capable of independent disinfection and sterilization. An instrument drive end is generally designed as non sterile. To ensure sterility during the surgical operations, a sterile adapter needs to be added between the instrument drive and the surgical instrument during surgery to isolate the non sterile instrument drive end and the sterile surgical instrument end during surgery.

In related technologies, the transmission and connection between the instrument drive and the sterile adapters mostly adopts a single point connection method, which requires a single protrusion on a transmission and connection member for the instrument drive to be orientated in the same direction as an orientation of a single recess on a transmission and connection member for the sterile adapter, and the protrusion and the recess is in one-to-one correspondence. The single point connection method requires a lot of actions during operation, and a certain gap between the protrusions and the recess during the matching to ensure assembly reliability. However, the gap can affect torque transmission and generate transmission clearance, resulting in a relatively low reliability.

Patent document WO2021037170A1 discloses a transmission assembly for a surgical instrument, comprising a first transmission disk and a second transmission disk, wherein : the first transmission disk has a second end, and the second transmission disk has a third end arranged face to face with the second end, one of an end face of the second end and an end face of the third end has a first guide surface formed thereon, and the other one of the end face of the second end and the end face of the third end is provided with a second engagement component, wherein at least one first engagement component is provided on the first guide surface, and the second engagement component is configured to be engageable with the first engagement component, and the second engagement component is configured to move along the first guide surface until the second engagement component is engaged with the first engagement component to allow a torque transfer between the first transmission disk and the second transmission disk.

Patent document WO2021137107A1 discloses a sterile adapter for coupling to an instrument device manipulator of a medical robotic system, the sterile adapter comprising: a housing having a manipulator interface and a tool interface configured to operatively couple the instrument device manipulator to a tool such that the sterile adapter transfers torque from the instrument device manipulator to the tool when the tool is coupled to the sterile adapter; and a sensor target coupled to the housing and movable relative to the manipulator interface for permitting detection of the tool that is coupled to the sterile adapter, wherein the sterile adapter is configured to form a sterile barrier between the tool interface and the manipulator interface.

Patent document WO2020069430A1 discloses a robotic medical system, comprising: an instrument drive mechanism comprising a drive output configured to rotate and engage a corresponding drive input on a handle of a robotic medical instrument, wherein the robotic medical instrument comprises a pre-tensioned pull wire actuated by the drive input, a motor associated with the drive output and configured to rotate the drive output, and a torque sensor associated with the drive output and configured to measure torque imparted on the drive output; and at least one computer-readable memory in communication with at least one processor, the memory having stored thereon computer-executable instructions that cause the at least one processor to activate the motor associated with the drive output to rotate the drive output in response to a torque signal from the torque sensor associated with the drive output.

Patent document WO2011037394A2 discloses a sterile adapter arranged at and formed on an instrument holder on an operation robotic arm and installed between a surgical instrument on the said instrument holder, comprising: a body portion having a first surface opposite the instrument holder and a second surface opposite the surgical instrument; a first wheel supported by the body portion and exposed to the first surface, the first wheel being mated to a first driving wheel formed in the instrument holder; a sterilized adapter including a second wheel that is supported by the body portion and exposed to the second surface, and is mated to a second drive wheel formed in the instrument holder.

Patent document WO2019108437A2 discloses an interface for use with a surgical system, the interface comprising: a proximal body portion configured to mechanically engage an instrument control unit of the surgical system; a distal body portion disposed in mechanical cooperation with the proximal body portion and being configured to mechanically engage an instrument drive assembly of the surgical system; a cavity defined between the proximal body portion and the distal body portion; a proximal coupler disposed at least partially within the cavity and being configured to engage a driving element of the instrument control unit; a distal coupler disposed at least partially within the cavity, a distal end of the distal coupler including a distally-facing mating surface having a plurality of protrusions configured to engage a driven element of the instrument drive assembly; and a biasing element disposed in mechanical cooperation with the proximal coupler and the distal coupler.

### SUMMARY

In a first aspect, a transmission and connection structure is provided according to the embodiments of the present application. The claimed solution is specified by the subject-matter according to claim 1, and dependent claims specify embodiments thereof.

### BRIEF DESCRIPTION OF THE DRAWINGS

The following drawings of the present application are herein used as a part of the present application for understanding the present application. Embodiments of the present application are described with reference to the accompanying drawings to explain the principles of the present application, in which:
FIG. 1 is a partial three-dimensional schematic view of a surgical robot;
FIG. 2 is an explosive view of the surgical robot shown in FIG. 1;
FIG. 3 is an explosive view of the surgical robot shown in FIG. 2 from another perspective;
FIG. 4 is a three-dimensional schematic view of a transmission and connection member for the instrument drive shown in FIG. 2;
FIG. 5 is a three-dimensional schematic view of the transmission and connection member for the instrument drive in FIG. 4 from another perspective;
FIG. 6 is a cross-sectional view of the transmission and connection member for the instrument drive shown in FIG. 4 along A-A';
FIG. 7 is a cross-sectional view of the transmission and connection member for the instrument drive shown in FIG. 4 along B-B';
FIG. 8 is a three-dimensional view of the transmission and connection member for the sterile adapter shown in FIG. 2;
FIG. 9 is a cross-sectional view of the transmission and connection member for the sterile adapter shown in FIG. 8 along C-C';
FIG. 10 is a cross-sectional view of the transmission and connection member for the sterile adapter shown in FIG. 8 along D-D';
FIG. 11 is a partial three-dimensional schematic view of a surgical robot provided according to a second optional embodiment of the present application which is not claimed;
FIG. 12 is a three-dimensional view of the transmission and connection member for the instrument drive shown in FIG. 11;
FIG. 13 is a front view of the transmission and connection member for the instrument drive shown in FIG. 12;
FIG. 14 is a three-dimensional view of the transmission and connection member for the sterile adapter shown in FIG. 11;
FIG. 15 is a three-dimensional view of the transmission and connection member for the sterile adapter shown in FIG. 14 from another perspective;
FIG. 16 is a front view of the transmission and connection member for the sterile adapter shown in FIG. 14; and
FIG. 17 is a cross-sectional view of the transmission and connection member for the sterile adapter shown in FIG. 14.

### DETAILED DESCRIPTION OF THE EMBODIMENTS

Detailed illustration is given in the following description to provide a more thorough understanding of the present application. However, it will be apparent to those skilled in the art that embodiments of the present application may be practiced without one or more of these details. In other examples, some technical features well known in the art are not described in order to avoid confusion with embodiments of the present application.

The terms referenced in the present application are only for describing specific embodiments, and are not intended to limit the exemplary embodiments according to the present application. As used here, unless the context otherwise clearly indicates, the singular form may also intended to include the plural form. In addition, in response to the terms "comprising" and/or "including" being referenced in the present application, they indicate the existence of the said features, entirety, step, operation, element, and/or component, but do not exclude the existence or addition of one or more other features, entirety, step, operation, element, component, and/or their combination. Ordinal numbers such as "first" and "second" referenced in the present application are merely identifiers and do not have any other meaning, e.g., does not represent a specific order. In addition, for example, the term "first component/member" does not imply the existence of a "second component/member", and the term "second component/member" does not imply the existence of the "first component/member".

The terms "up", "down", "front", "rear", "left", "right", and similar expressions referenced in the present application are merely intended to explain the present application rather than limit the present application.

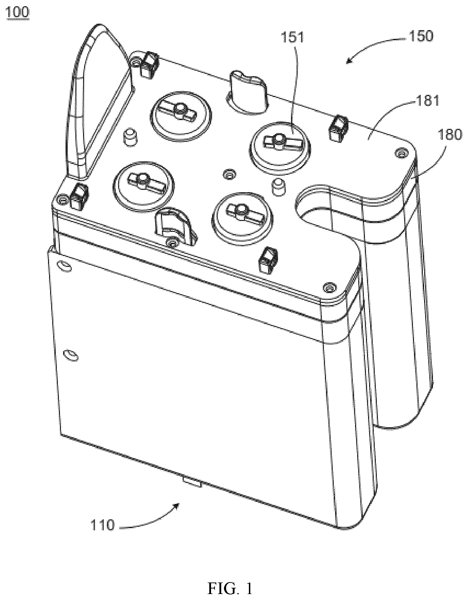

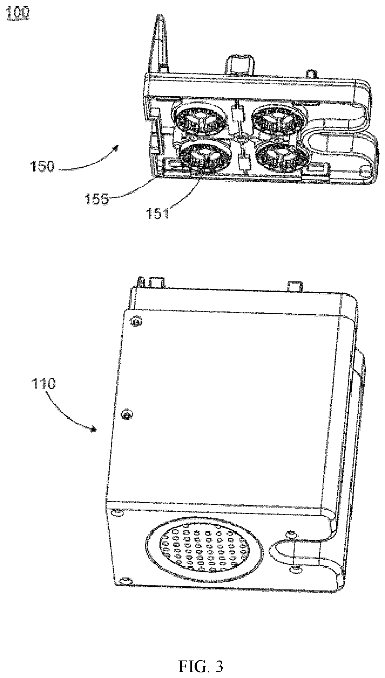

Referring first to FIG. 1, FIG. 2, and FIG. 3, a surgical robot 100 includes a surgical instrument (not shown), a sterile adapter 150, and an instrument drive 110. The sterile adapter 150 is connected to the instrument drive 110, and the surgical instrument is connected to the sterile adapter 150. Specifically, a lower surface of the sterile adapter 150 is connected to an upper surface of the instrument drive 110. A front-end of the surgical instrument is configured as surgical tools such as forceps, scissors, clamps, etc. A rear end of the surgical instrument is connected to an upper surface of the sterile adapter 150, and the instrument drive 110 is configured to provide drive force to the instrument executing mechanism in the middle of the rear end of the surgical instrument via the sterile adapter 150, so that the pulling components (e.g., wires or ropes) in a shaft enable the above surgical tools to complete movement such as pitching, deflection, and gripping. A surface of instrument drive 110 is engaged with the lower surface of sterile adapter 150. A rear end surface of the surgical instrument is engaged with the upper surface of sterile adapter 150. During operation, the surface of instrument drive 110, the lower surface of sterile adapter 150, the upper surface of sterile adapter 150, and the rear end surface of the surgical instrument do not change in relative position. The transmission and connection member 111 for the instrument drive 110, the transmission and connection member 151 for the sterile adapter 150, and a transmission and connection member for the surgical instrument at the rear end are connected and fixed in specific ways, respectively, so that during operation, the instrument drive 110 is configured to drive the transmission and connection member 111 for the instrument drive 110 to rotate and transmit the driving force (or torque) to the transmission and connection member for the surgical instrument at the rear end via the transmission and connection member 151 for the sterile adapter 150, thereby achieving control of the surgical tools. The transmission and connection structure is described in this embodiment.

The instrument drive 110 includes a transmission and connection member 111 for the instrument drive 110, the sterile adapter 150 includes a transmission and connection member 151 for the sterile adapter 150, the transmission and connection member 151 for the sterile adapter 150 is connected to the transmission and connection member 111 for the instrument drive 110, and the transmission and connection member for the surgical instrument at the rear end is connected to the transmission and connection member 151 for the sterile adapter 150.

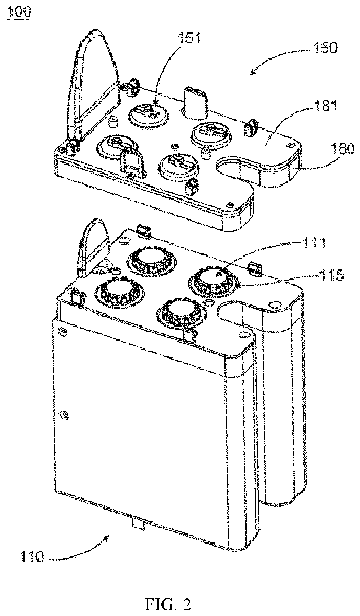

Specifically, referring to FIG. 2, FIG. 3, FIG. 4, FIG. 5, and FIG. 8, the transmission and connection member 111 for the instrument drive 110 has a first drive end 112 and a first connection end 113. The first drive end 112 is arranged away from the sterile adapter 150 and is configured to connect to a drive component (not shown, may be a motor) inside the instrument drive 110. The first connection end 113 is close to the sterile adapter 150 and is configured to be coupled with the transmission and connection member 151 for the sterile adapter 150.

The transmission and connection member 151 for the sterile adapter 150 has a second drive end 152 and a second connection end 153. The second connection end 153 is close to the instrument drive 110 and is configured to be coupled with the transmission and connection member 111 for the instrument drive 110. The second drive end 152 is arranged away from the instrument drive 110 and is configured to be coupled with the transmission and connection member for the surgical instrument at the rear end. The transmission and connection member 111 for the instrument drive 110 further has a first toothed portion 115 arranged at the first connection end 113. The transmission and connection member 151 for the sterile adapter 150 further has a second toothed portion 155 arranged at the second connection end 153. Moreover, the second toothed portion 155 is configured to engage with the first toothed portion 115.

Therefore, according to the surgical instrument of the present application, the meshing of two toothed structures can achieve connection between the transmission and connection member 151 for the sterile adapter 150 and the transmission and connection member 111 for the instrument drive 110 at any point around the entire circumference, and the meshing connection is tight without gaps, thereby improving the stability and accuracy of the transmission.

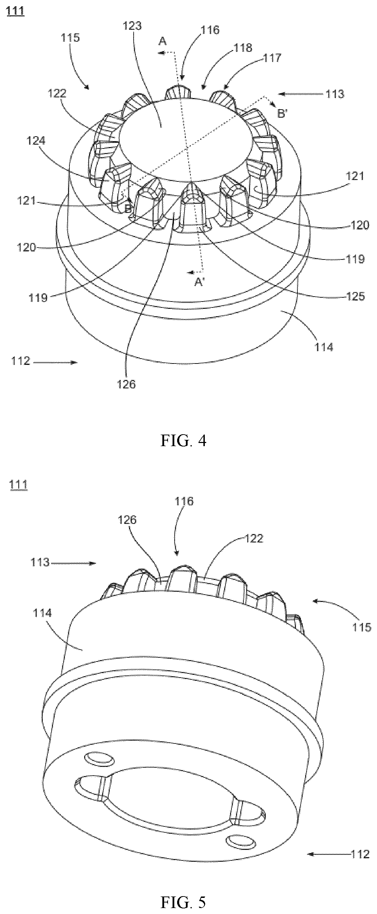

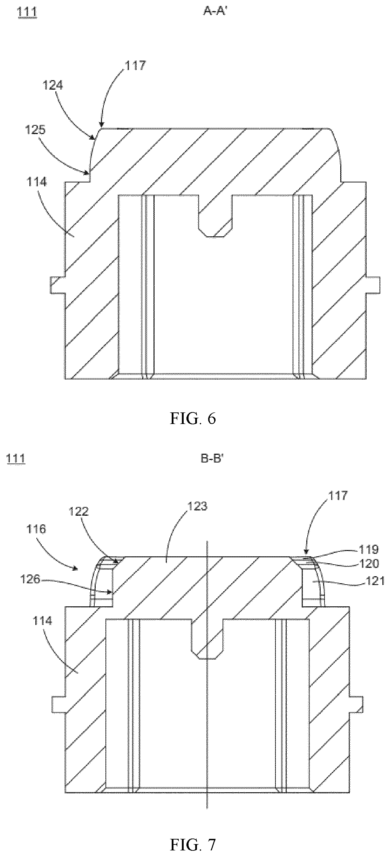

The following will provide a detailed explanation of the transmission and connection member 111 for the instrument drive 110 in conjunction with FIG. 4 to FIG. 7. The transmission and connection member 111 for the instrument drive 110 has a first body portion 114 and a positioning portion protruding from the first body portion 114. Therefore, in this embodiment, the positioning portion is configured as a protruding portion 123. The end of the positioning portion forms the first connection end 113. The first toothed portion 115 is arranged at the top of the first body portion 114 and along the circumference of the first body portion 114. Specifically, the first toothed portion 115 is arranged around the protruding portion 123. The first body portion 114 and the positioning portion may both be optionally configured as a cylindrical shape.

The first toothed portion 115 includes multiple first teeth 116, which can be odd or even in number. The multiple first teeth 116 are uniformly arranged along the circumference of the transmission and connection member 111 for the instrument drive 110, and each of the multiple first teeth 116 is connected to the protruding portion 123.

Each of the multiple first teeth 116 has the same structure, and each of the multiple first teeth 116 is symmetrical relative to the axial section of the first body portion 114. That is, each of the multiple first teeth 116 is symmetrical about its own central plane as the plane of symmetry, which is conducive to direct installation, thereby making installation convenient and reducing the alignment operations between the transmission and connection member 111 for the instrument drive 110 and the transmission and connection member 151 for the sterile adapter 150.

Each of the multiple first teeth 116 has a first tooth peak 117, and a first tooth trough 118 is formed between two adjacent first teeth 116. On both sides of each of the multiple first teeth 116, a pair of symmetrical first guide surfaces 119, a pair of symmetrical first transition surfaces 120, and a pair of symmetrical first engaging surfaces 121 are sequentially arranged along the direction from the first tooth peak 117 to the root of the respective one of the multiple first teeth 116.

The pair of first guide surfaces 119 meet at the first tooth peak 117, or specifically, each first guide surface 119 is inclined towards the first tooth peak 117, and is formed as a flat surface, which has an inclined angle, or the first guide surface 119 is formed as a smoothly curved surface, so that the first tooth peak 117 is formed as a sharp corner or a rounded corner. Therefore, the radial cross-sectional area (or radial cross-sectional width) of each of the multiple first teeth 116 at the first tooth peak 117 is smaller than the radial cross-sectional area (or radial cross-sectional width) of each of the multiple first teeth 116 at the root of the respective one of the multiple first teeth 116, resulting in each of the multiple first teeth 116 forming a shape with one end wide and one end narrow.

The pair of first engaging surfaces 121 extend along a height direction of each of the multiple first teeth 116 to the first body portion 114. The pair of first engaging surfaces 121 are formed as vertical surfaces or slightly inclined surfaces. The first engaging surface 121 is configured to be in contact and mesh with the second tooth 156 to transmit torque. The first engaging surface 121 can transmit torque to the maximum extent when formed as a vertical surface. The first engaging surface 121 may also be formed as a slightly inclined surface, which can seek a balance between transmitting torque and accuracy requirements. In addition, the slightly inclined structure of the first engaging surface 121 can achieve a highly accurate fit when matched with the corresponding matching surface of the each of the multiple second teeth 116. Specifically, a slightly inclined surface can refer to a surface inclined by an angle within 15 degrees from the vertical direction. Optionally, the included angle between the slightly inclined surface and the vertical direction is less than or equal to 5 degrees.

The first transition surface 120 is connected between the first guide surface 119 and the first engaging surface 121 to serve as a transition therebetween and perform guiding. The inclined degree of the first transition surface 120 is greater than that of the first guide surface 119, thereby achieving a gentle transition from the first guide surface 119 to the first engaging surface 121.

The protruding portion 123 includes a first matching inclined surface 122 and a first matching cylindrical surface 126 both extending along the circumference of the protruding portion 123. The first matching inclined surface 122 is formed at the top portion of the protruding portion 123 and extends along a circumference of the protruding portion 123, and a radial cross-sectional diameter of the first matching inclined surface 122 gradually increases along a direction from the first tooth peak 117 to the root of the respective one of the multiple first teeth 116. In other words, the first matching inclined surface 122 can be formed as a straight inclined surface or an arc inclined surface, such as a rounded corner or a chamfer. The first matching cylindrical surface 126 is adjacent to the first matching inclined surface 122. The first matching cylindrical surface 126 and the first matching inclined surface 122 mentioned above can be formed as connecting surfaces between the two first teeth 116 on the protruding portion 123.

Referring to FIG. 4, FIG. 5, and FIG. 6, an outer circumferential surface of the first toothed portion 115 arranged at an outer circumferential side of each of the multiple first teeth 116 transits from the first smooth arc surface 124 to a first cylindrical surface 125 along the direction from the first tooth peak 117 to the root of the respective one of the multiple first teeth 116. The first smooth arc surface 124 gradually increases in radial cross-sectional radius or radial cross-sectional diameter along a direction from the first tooth peak 117 to the first cylindrical surface 125. Therefore, the first smooth arc surface 124 substantially forms a shape inclined towards the middle of the transmission and connection member 111 for the instrument drive 110. Specifically, the radial cross-sectional radius or radial cross-sectional diameter of the circumference of the first smooth arc surface 124 gradually increases from the first tooth peak 117 to the root of the respective one of the multiple first teeth 116. The first cylindrical surface 125 extends from the first smooth arc surface 124 to the first body portion 114.

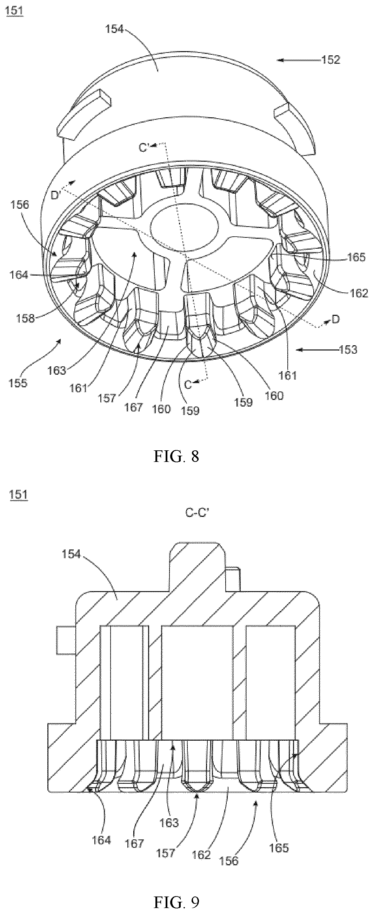

The transmission and connection member 151 for the sterile adapter 150 will be described in detail below in conjunction with FIG. 8, FIG. 9, and FIG. 10. The transmission and connection member 151 for the sterile adapter 150 has a second body portion 154, which is recessed inward at the second connection end 153 to form a matching portion. The matching portion shown in the figure is in the shape of a cover and can be engaged with the first connection end. Therefore, in this embodiment, the matching portion may also be referred to as the recessing portion 163. The recessing portion 163 is opened at the second connection end 153, and the recessing portion 163 is configured to accommodate the protruding portion 123 of the transmission and connection member 111 for the instrument drive 110, so that the protruding portion 123 can at least partially extend into the recessing portion 163.

The second toothed portion 155 is arranged at the bottom of the second body portion 154 and is arranged along the circumference of the second body portion 154. Specifically, the second toothed portion 155 is arranged within the recessing portion 163 and is located at the opening of the recessing portion 163. The second toothed portion 155 includes multiple second teeth 156 uniformly arranged along the circumference of the transmission and connection member 151 for the sterile adapter 150. Each of the multiple second teeth 156 has the same structure, and is symmetrical relative to the axial section of the second body portion 154.

Each of the multiple second teeth 116 has a second tooth peak 157, and a second tooth trough 158 is formed between two adjacent second teeth 156. In engagement of the first toothed portion 115 with the second toothed portion 155, each of the multiple first teeth 116 extends into the second toothed trough 158, and the each of the multiple second teeth 116 extends into the first toothed trough 118. In other words, in response to the transmission and connection member 111 for the instrument drive 110 being connected to the transmission and connection member 151 for the sterile adapter 150, the protruding portion 123 is matched with the recessing portion 163, and the first toothed portion 115 is matched with the second toothed portion 155, so that the transmission and connection member 111 for the instrument drive 110 and the transmission and connection member 151 for the sterile adapter 150 can be tightly connected at any position.

The each of the multiple second teeth 116 includes a second guide surface 159 corresponding to the first guide surface 119, a second transition surface 160 corresponding to the first transition surface 120, a second engaging surface 161 corresponding to the first engaging surface 121, a second smooth arc surface 164 similar to the first smooth arc 124, and a second cylindrical surface 165 similar to the first cylindrical surface 125.

Specifically, a pair of second guide surfaces 159 are engaged at the second tooth peak 157, or, the second guide surface 159 is inclined towards the second tooth peak 157, and is formed as a straight surface (straight inclined surface) or a smooth arc surface, so that the second tooth peak 157 is formed as a sharp corner or a rounded corner.

Therefore, the radial cross-sectional area of the each of the multiple second teeth 116 at the second tooth peak 157 is smaller than the radial cross-sectional area of the each of the multiple second teeth 116 at the root of the respective one of the multiple second teeth 116, resulting in the each of the multiple second teeth 116 forming a shape with one end wide and one end narrow. Moreover, both the first tooth peak 117 and the second tooth peak 157 are formed with sharp corners or rounded corners to have a shape with one end wide and one end narrow, which can avoid the first tooth peak 117 and the second tooth peak 157 from being in contact with each other during docking, thereby allowing the first tooth peak 117 to be quickly stagger with the second tooth peak 157 and facilitating rapid installation.

A pair of second engaging surfaces 161 extends along the height direction of the each of the multiple second teeth 116 to the second body portion 154, and the pair of second engaging surfaces 161 are formed as vertical surfaces or slightly inclined surfaces. The second engaging surface 161 is configured to be in contact and mesh with the first engaging surface 121 of each of the multiple first teeth 116.

The second transition surface 160 is connected between the second guide surface 159 and the second engaging surface 161 to serve as a transition therebetween and perform guiding. The inclined degree of the second transition surface 160 is greater than that of the second guide surface 159, thereby achieving a gentle transition from the second guide surface 159 to the second engaging surface 161.

A second matching inclined surface 162 and a second matching cylindrical surface 167 are arranged on the inner wall of the recessing portion 163. The second matching inclined surface 162 is arranged at the circumference of the recessing portion 163 at the second connection end 153, and a radial cross-sectional diameter of the second matching inclined surface 162 gradually decreases along the direction from the second tooth peak 157 to the root of the respective one of the multiple second teeth 116. In other words, the second matching inclined surface 162 can be optionally formed as a straight inclined surface or an arc inclined surface, such as a rounded corner or a chamfer. The second matching cylindrical surface 167 is adjacent to the second matching inclined surface 162. The second matching cylindrical surface 167 and the second matching inclined surface 162 mentioned above can be formed as a connecting surface between the two adjacent second teeth 156 on the inner wall of the recessing portion 163.

A second smooth arc surface 164 and a second cylindrical surface 165 are sequentially arranged from top to bottom on the inner side along the circumference of the each of the multiple second teeth 116. The second smooth arc surface 164 is formed as a smooth arc surface and extends to the second tooth peak 157, thereby connecting with a pair of second guide surfaces 159 and a pair of second transition surfaces 160. As a result, the second smooth arc surface 164 substantially forms a shape inclined towards the outer circumference of the transmission and connection member 151 for the sterile adapter 150. Specifically, the radial cross-sectional radius or radial cross-sectional diameter of the circumference of the second smooth arc surface 164 gradually increases along the direction from the second tooth peak 157 to the root of the respective one of the multiple second teeth 116. The second cylindrical surface 165 extends from the second smooth arc surface 164 to the second body portion 154.

Therefore, in response to the transmission and connection member 111 for the instrument drive 110 being connected to the transmission and connection member 151 for the sterile adapter 150, the first smooth arc surface 124 can be guided in conjunction with the second matching inclined surface 162, and the second smooth arc surface 164 can be guided in conjunction with the first matching inclined surface 122, which makes it easier and smoother for the protruding portion 123 to enter the recessing portion 163. Moreover, the first guide surface 119 can be guided in conjunction with the second guide surface 159, which makes it easier for each of the multiple first teeth 116 to enter the second tooth trough 158 and the each of the multiple second teeth 116 to enter the first tooth trough 118. Thus, the first cylindrical surface 125 of each of the multiple first teeth 116 can form an assembly fit (or mesh) with the second matching cylindrical surface 167, and the second cylindrical surface 165 of the each of the multiple second teeth 116 can form an assembly fit (or mesh) with the first matching cylindrical surface 126.

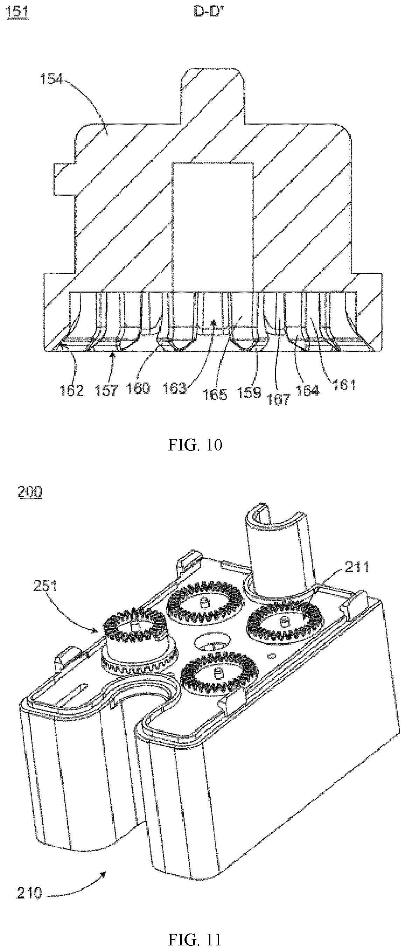

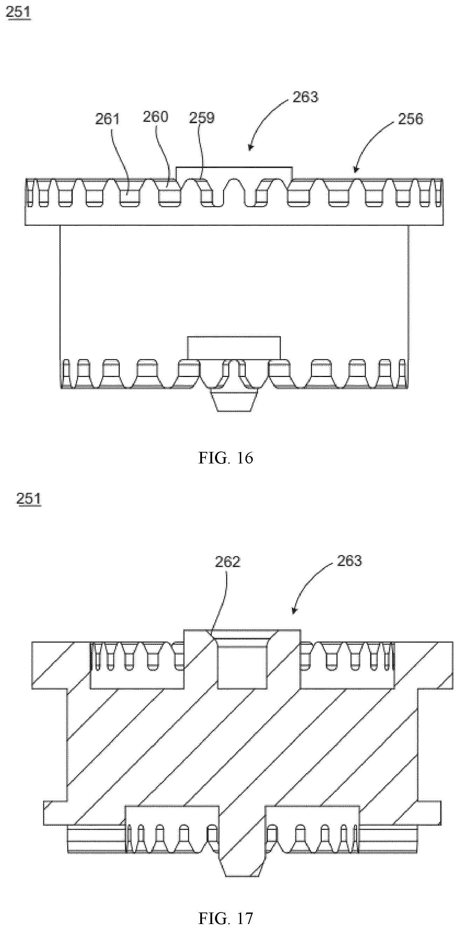

According to the second optional embodiment of the present application which is not claimed, referring to FIG. 11 to 17, a main structure of an instrument drive 210 and a sterile adapter (not shown) of the second optional embodiment is the same as that of the first embodiment, and a main difference is in the transmission and connection member 211 for the instrument drive 210 and the transmission and connection member 251 for the sterile adapter.

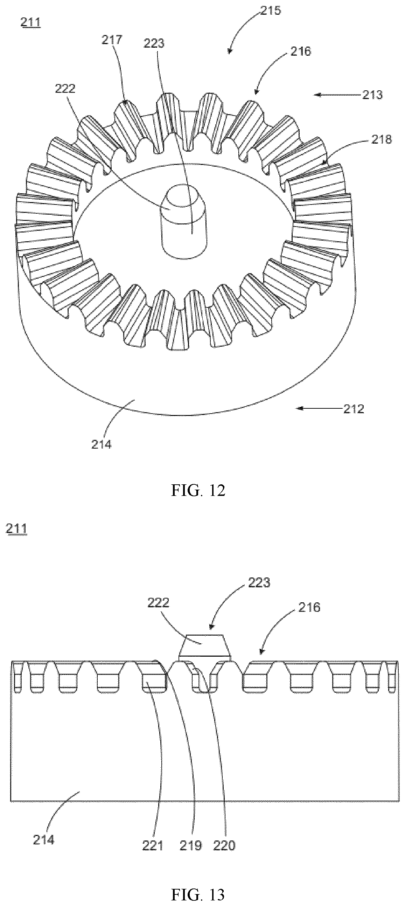

Specifically, FIG. 12 and FIG. 13 illustrate the transmission and connection member 211 for the instrument drive 210 of the second optional embodiment of the present application. The transmission and connection member 211 for the instrument drive 210 has a first drive end 212 and a first connection end 213, which are similar to the first embodiment and will not be further described here

The transmission and connection member 211 for the instrument drive 210 further includes a first body portion 214 and a first toothed portion 215 arranged at the edge of the first body portion 214 along a circumferential direction, and the first toothed portion 215 is continuous in a circumferential direction and is located at the first connection end 213. The structure of each of the multiple first teeth 216 may be identical and symmetrical (axial symmetrical).

The first tooth 216 further includes a first tooth peak 217, a first tooth trough 218, a pair of first guide surfaces 219, a pair of first transition surfaces 220, and a pair of first engaging surfaces 221 similar to the first tooth peak 117, the first tooth trough 118, the pair of first guide surfaces 119, the pair of first transition surfaces 120, and the pair of first engaging surfaces 121 in the first embodiment. The difference between the first tooth 216 and the first tooth 116 in the first embodiment is that there is no matching surfaces in the outer circumference of the first tooth 216, and assembly is only achieved by relying on the engaging surfaces. The first tooth 216 is formed to extend inward from the circumference of the first body portion 214.

The transmission and connection member 211 for the instrument drive 210 further includes a positioning portion configured as a positioning column 223, which protrudes from the middle of the first body portion 214 at the first connection end 113. In addition, the multiple first teeth 216 are not arranged on the positioning column 223 but are spaced from the positioning column 223. A first matching inclined surface 222 is formed on a top portion of the positioning column 223 along the circumference of the positioning column 223.

In addition, the width of each of the multiple first tooth 216 along the circumferential direction gradually decreases in a radial direction towards the positioning column 223, which can easily cause a partial top surface to be formed on the first tooth peak 217. For example, the outer circumferential side of the first tooth peak 217 shown in FIG. 12 has a top surface, and a sharp corner or a rounded corner is formed on the inner outer circumferential side of the first tooth peak 217. Optionally, the first tooth peak 217 is integrally formed with the sharp corner or the rounded corner.

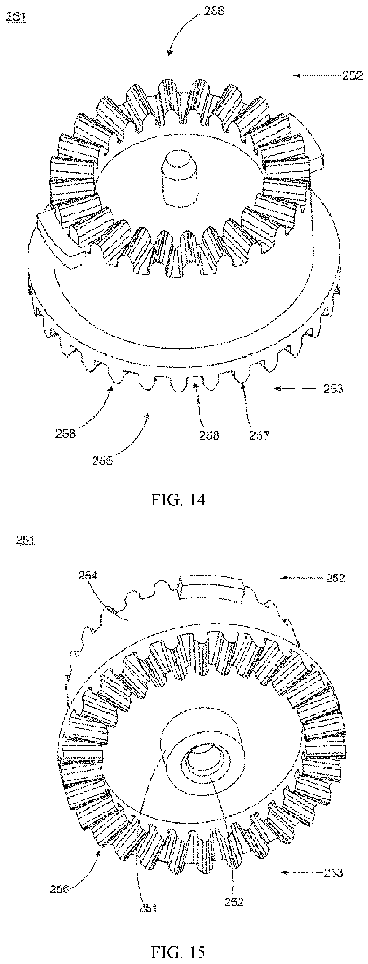

FIG. 14, FIG. 15, FIG. 16, and FIG. 17 illustrate the transmission and connection member 251 for the sterile adapter in the second optional embodiment of the present application.

The transmission and connection member 251 for the sterile adapter has a second drive end 252 and a second connection end 253, which are similar to the second drive end 152 and the second connection end 153 of the transmission and connection member 151 for the sterile adapter 150 in the first embodiment and will not be further described here. The transmission and connection member 251 for the sterile adapter further includes a second body portion 254 and a second toothed portion 255 arranged on the edge of the second body portion 254 along a circumferential direction of the second body portion 254. The second toothed portion 255 is arranged at the second connection end 253 and is continuous along circumferential direction. The structure of each of the multiple second teeth 256 may be identical and symmetrical (axial symmetrical).

The second tooth 256 further includes a second tooth peak 257, a second tooth trough 258, a pair of second guide surfaces 259, a pair of second transition surfaces 260, and a pair of second engaging surfaces 261 similar to the second tooth peak 157, the second tooth trough 158, the pair of second guide surfaces 159, the pair of second transition surfaces 160, and the pair of second engaging surfaces 161 in the first embodiment. The difference between the second tooth 256 and the second tooth 156 in the first embodiment is that there is no matching surfaces on the outer circumference of the second tooth 256. The second tooth 256 is configured to extend along a radial direction of the second body portion 254, and the multiple second teeth 256 are configured to extend over the outer circumference of the second body portion 254. That is, a diameter of the second toothed portion 255 is greater than a diameter of a part of the second body portion 254 close to the second connection end 253.

The transmission and connection member 251 for the sterile adapter further includes a matching portion configured as a positioning cylinder 263 arranged at the second connection end 253 of the second body portion 254 and protrudes from the middle of the second body portion 254. In addition, the multiple second tooth 256 are spaced from the positioning cylinder 263. A second matching inclined surface 262 is formed on a circumference of a top portion of an inner wall of the positioning cylinder 263. The shape of the positioning cylinder 263 matches the shape of the positioning column 223, so that in response to the transmission and connection member 211 for the instrument drive 210 being connected to the transmission and connection member 251 for the sterile adapter, the positioning column 223 extends into the positioning cylinder 263, and the first toothed portion 215 is engaged with the second toothed portion 255.

In addition, a width of the second tooth 256 along a circumferential direction of the second tooth 256 gradually decreases in a radial direction towards the positioning cylinder 263, which can easily cause a partial top surface to be formed at the second tooth peak 257. For example, the outer circumferential side of the second tooth peak 257 shown in FIG. 15 has a top surface, and a sharp corner or a rounded corner is formed at the inner outer circumferential side of the second tooth peak 257. Optionally, the second tooth peak 257 is integrally formed with the sharp corner or the rounded corner.

In the illustrated optional embodiment, a toothed portion may be further arranged on the second drive end 252 of the transmission and connection member 251 for the sterile adapter. For example, the toothed portion is referred to as a third toothed portion 266 (as shown in FIG. 14). The third toothed portion 266 may have the same structure as the second toothed portion 255, except for that third toothed portion 266 has a diameter different from the second toothed portion 255, and will not be further described here. The third toothed portion 266 is configured to mesh with the transmission and connection member for the surgical instrument at the rear end (not shown) of the surgical instrument, in order to further achieve the technical effect of smooth installation in all directions without the need for alignment.

Unless otherwise defined, technical and scientific terms used herein have the same meanings as are commonly understood by those skilled in the art of the present application. Terms used herein are for specific practical purposes only and are not intended to limit the present application. Features described herein in one embodiment may be applied to another embodiment alone or in combination with other features unless the features are not applicable in the other embodiment or otherwise noted.

## Claims

1. A transmission and connection structure, comprising:
an instrument drive (110) comprising a transmission and connection member (111) for the instrument drive (110), wherein the transmission and connection member (111) for the instrument drive (110) has a first connection end (113) configured to be coupled to a sterile adapter (150), the first connection end (113) is provided with a first toothed portion (115), and the first toothed portion (115) comprises a plurality of first teeth (116) evenly spaced and arranged along a circumference of the transmission and connection member (111) for the instrument drive (110); and
the sterile adapter (150) comprising a transmission and connection member (151) for the sterile adapter (150), wherein the transmission and connection member (151) for the sterile adapter (150) has a second connection end (153) configured to be coupled to the first connection end (113) of the instrument drive (110), the second connection end (153) is provided with a second toothed portion (155), and the second toothed portion (155) comprises a plurality of second teeth (156) evenly spaced and arranged along a circumference of the transmission and connection member (151) for the sterile adapter (150);
during operation, the instrument drive (110) is configured to drive the transmission and connection member (111) for the instrument drive (110) to rotate and transmit a torque to a surgical instrument via the transmission and connection member (151) for the sterile adapter (150),
wherein the second toothed portion (155) is configured to mesh with the first toothed portion (115) to transmit the torque via the plurality of first teeth (116) and the plurality of second teeth (156),
each of the plurality of first teeth (116) has a first tooth peak (117) formed as a sharp corner or a rounded corner; and
each of the plurality of second teeth (156) has a second tooth peak (157) formed as a sharp corner or a rounded corner;
the transmission and connection member (111) for the instrument drive (110) further comprises a first body portion (114) and a positioning portion (123) disposed at the first connection end (113) and protruding from the first body portion (114), the first toothed portion (115) protrudes from an outer peripheral surface of the positioning portion and is arranged around the positioning portion, the outer peripheral surface comprises a first matching inclined surface (122) extending along a circumference of a top portion of the positioning portion,
wherein each of the plurality of first teeth (116) has a first smooth arc surface (124) at a periphery of the first toothed portion (115), and the first smooth arc surface (124) has a diameter in a radial cross-section of the first toothed portion (115), the diameter of the first smooth arc surface (124) gradually increases along a direction from a first tooth peak (117) to a root of each of a respective first tooth (116);
wherein the transmission and connection member (151) for the sterile adapter (150) further comprises a second body portion (154) which is recessed inward at the second connection end (153) to form a matching portion (163), the second toothed portion (155) protrudes from an inner peripheral surface of the matching portion and is arranged around the matching portion, the inner peripheral surface comprises a second matching inclined surface (162) extending along a circumference of a bottom portion of the matching portion;
wherein each of the plurality of second teeth (156) has a second smooth arc surface (164) at a periphery of the second toothed portion (155) , and the second smooth arc surface (164) has a diameter in a radial cross-section of the second toothed portion (155), the diameter of the second smooth arc surface (164) gradually decreases along a direction from a second tooth peak (157) to a root of each of a respective second tooth (156);
wherein the first smooth arc surface (124) is guided in conjunction with the second matching inclined surface (162), and the second smooth arc surface (164) is guided in conjunction with the first matching inclined surface (122).

2. The transmission and connection structure according to claim 1, wherein each of the plurality of first teeth (116) is symmetrical about a respective centerline, and each of the plurality of second teeth (156) is symmetrical about a respective centerline.

3. The transmission and connection structure according to claim 1, wherein each of the plurality of first teeth (116) has two first guide surfaces (119) arranged symmetrically by two sides of the first tooth peak (117), respectively, and a horizontal distance between the two first guide surfaces (119) gradually increases in a direction away from the first tooth peak (117), and each of the two first guide surfaces (119) is formed as a flat surface or a smoothly curved surface; and
each of the plurality of second teeth (156) has two second guide surfaces (159) arranged symmetrically by two sides of the second tooth peak (157), respectively, and a horizontal distance between the two second guide surfaces (159) gradually increases in a direction away from the second tooth peak (157), and each of the two second guide surfaces (159) is formed as a flat surface or a smoothly curved surface.

4. The transmission and connection structure according to any one of claims 1 to 3, wherein each of the plurality of first teeth (116) has two first engaging surfaces (121) arranged symmetrically on two sides of a respective first tooth (116), and each of the two first engaging surfaces (121) is formed as a vertical surface or a slightly inclined surface;
each of the plurality of second teeth (156) has two second engaging surfaces (161) arranged symmetrically on two sides of a respective second tooth (156), and each of the two second engaging surfaces (161) is formed as a vertical surface or a slightly inclined surface;
in engagement of the first toothed portion (115) with the second toothed portion (155), each of the two first engaging surfaces (121) is in contact with a corresponding second engaging surface (161).

5. The transmission and connection structure according to claim 1 or 3, wherein each of the plurality of first teeth (116) has a width in a radial cross-section at the first tooth peak (117) smaller than a width in a radial cross-section at a root of a respective first tooth (116); and/or
each of the plurality of second teeth (156) has a width in a radial cross-section at the second tooth peak (157) smaller than a width in a radial cross-section at a root of a respective second tooth (156).

6. The transmission and connection structure according to claim 1, wherein
the second toothed portion (155) has a plurality of connecting surfaces and each of which is arranged between two respective adjacent second teeth (156) of the second toothed portion (155), each of the plurality of connecting surfaces connects two respective engaging surfaces each corresponding to one of the respective two adjacent second teeth (156), and each of the plurality of connecting surfaces is partly formed as the second matching inclined surface (162), the second matching inclined surface (162) has a diameter in a radial cross-section of the second toothed portion (155), the diameter of the second matching inclined surface (162) gradually decreases along a direction from a second tooth peak (157) to a root of each of the plurality of second teeth (156), and the second matching inclined surface (162) is configured to slidably fit with the first smooth arc surface (124) at the periphery of the first toothed portion (115).

7. The transmission and connection structure according to claim 6, wherein each of the first teeth (116) has a first cylindrical surface (125) at a periphery of the first toothed portion (115), the first cylindrical surface (125) is a physical surface of the respective first tooth (116), and the first cylindrical surface (125) is adjacent to the first smooth arc surface (124) and extends to the root of each of the plurality of first teeth (116); and
each of the plurality of connecting surfaces is partly formed as a second matching cylindrical surface (167) adjacent to the second matching inclined surface (162), and the second matching cylindrical surface (167) is configured to fit with the first cylindrical surface (125) of the first toothed portion (115).

8. The transmission and connection structure according to claim 1, wherein the matching portion is arranged to correspond to the positioning portion, and the positioning portion extends at least partially into the matching portion.

9. The transmission and connection structure according to claim 8, wherein the transmission and connection member (151) for the sterile adapter (150) further comprises a second drive end (152) opposite to the second connection end (153).

10. A surgical robot (100), comprising a transmission and connection structure according to any one of claims 1 to 9 and a surgical instrument, wherein the sterile adapter (150) is connected to the instrument drive (110), and the surgical instrument is connected to the sterile adapter (150).

## Patentansprüche

1. Übertragungs- und Verbindungsstruktur, umfassend:
einen Instrumentenantrieb (110) mit einem Übertragungs- und Verbindungselement (111) für den Instrumentenantrieb (110), wobei das Übertragungs- und Verbindungselement (111) für den Instrumentenantrieb (110) ein erstes Verbindungsende (113) aufweist, das zur Kopplung mit einem sterilen Adapter (150) konfiguriert ist, das erste Verbindungsende (113) mit einem ersten Zahnabschnitt (115) versehen ist und der erste Zahnabschnitt (115) eine Vielzahl von ersten Zähnen (116) umfasst, die gleichmäßig beabstandet und entlang eines Umfangs des Übertragungs- und Verbindungselements (111) für den Instrumentenantrieb (110) angeordnet sind; und
der sterile Adapter (150) ein Übertragungs- und Verbindungselement (151) für den sterilen Adapter (150) umfasst, wobei das Übertragungs- und Verbindungselement (151) für den sterilen Adapter (150) ein zweites Verbindungsende (153) aufweist, das so konfiguriert ist, dass es mit dem ersten Verbindungsende (113) des Instrumentenantriebs (110) gekoppelt werden kann, das zweite Verbindungsende (153) mit einem zweiten Zahnabschnitt (155) versehen ist und der zweite Zahnabschnitt (155) eine Vielzahl von zweiten Zähnen (156) umfasst, die gleichmäßig beabstandet und entlang eines Umfangs des Übertragungs- und Verbindungselements (151) für den sterilen Adapter (150) angeordnet sind;
während des Betriebs ist der Instrumentenantrieb (110) so konfiguriert, dass er das Übertragungs- und Verbindungselement (111) für den Instrumentenantrieb (110) antreibt, um sich zu drehen und ein Drehmoment über das Übertragungs- und Verbindungselement (151) für den sterilen Adapter (150) auf ein chirurgisches Instrument zu übertragen,
wobei der zweite Zahnabschnitt (155) so konfiguriert ist, dass er mit dem ersten Zahnabschnitt (115) in Eingriff kommt, um das Drehmoment über die mehreren ersten Zähne (116) und die mehreren zweiten Zähne (156) zu übertragen,
jeder der mehreren ersten Zähne (116) eine erste Zahnspitze (117) aufweist, die als scharfe Ecke oder abgerundete Ecke ausgebildet ist; und
jeder der mehreren zweiten Zähne (156) eine zweite Zahnspitze (157) aufweist, die als scharfe Ecke oder abgerundete Ecke ausgebildet ist;
das Übertragungs- und Verbindungselement (111) für den Instrumentenantrieb (110) ferner einen ersten Körperabschnitt (114) und einen Positionierungsabschnitt (123) umfasst, der am ersten Verbindungsende (113) angeordnet ist und aus dem ersten Körperabschnitt (114) herausragt, der erste Zahnabschnitt (115) ragt aus einer Außenumfangsfläche des Positionierungsabschnitts heraus und ist um den Positionierungsabschnitt herum angeordnet, die Außenumfangsfläche umfasst eine erste passende Schrägfläche (122), die sich entlang eines Umfangs eines oberen Abschnitts des Positionierungsabschnitts erstreckt,
wobei jeder der mehreren ersten Zähne (116) eine erste glatte Bogenfläche (124) an einem Umfang des ersten Zahnabschnitts (115) aufweist und die erste glatte Bogenfläche (124) einen Durchmesser in einem radialen Querschnitt des ersten Zahnabschnitts (115) aufweist, wobei der Durchmesser der ersten glatten Bogenfläche (124) entlang einer Richtung von einer ersten Zahnspitze (117) zu einer Zahnwurzel jedes jeweiligen ersten Zahns (116) allmählich zunimmt;
wobei das Übertragungs- und Verbindungselement (151) für den sterilen Adapter (150) ferner einen zweiten Körperabschnitt (154) umfasst, der am zweiten Verbindungsende (153) nach innen vertieft ist, um einen Passabschnitt (163) zu bilden, wobei der zweite gezahnte Abschnitt (155) von einer Innenumfangsfläche des Passabschnitts vorsteht und um den Passabschnitt herum angeordnet ist, wobei die Innenumfangsfläche eine zweite passende geneigte Fläche (162) umfasst, die sich entlang eines Umfangs eines Bodenabschnitts des Passabschnitts erstreckt;
wobei jeder der mehreren zweiten Zähne (156) eine zweite glatte Bogenfläche (164) an einem Umfang des zweiten Zahnabschnitts (155) aufweist und die zweite glatte Bogenfläche (164) einen Durchmesser in einem radialen Querschnitt des zweiten Zahnabschnitts (155) aufweist, wobei der Durchmesser der zweiten glatten Bogenfläche (164) entlang einer Richtung von einer zweiten Zahnspitze (157) zu einer Zahnwurzel jedes jeweiligen zweiten Zahns (156) allmählich abnimmt;
wobei die erste glatte Bogenfläche (124) in Verbindung mit der zweiten passenden geneigten Fläche (162) geführt wird und die zweite glatte Bogenfläche (164) in Verbindung mit der ersten passenden geneigten Fläche (122) geführt wird.

2. Die Übertragungs- und Verbindungsstruktur gemäß Anspruch 1, wobei jeder der mehreren ersten Zähne (116) symmetrisch zu einer jeweiligen Mittellinie ist und jeder der mehreren zweiten Zähne (156) symmetrisch zu einer jeweiligen Mittellinie ist.

3. Die Übertragungs- und Verbindungsstruktur gemäß Anspruch 1, wobei jeder der mehreren ersten Zähne (116) zwei erste Führungsflächen (119) aufweist, die symmetrisch zu zwei Seiten des ersten Zahnkamms (117) angeordnet sind, und wobei ein horizontaler Abstand zwischen den beiden ersten Führungsflächen (119) in einer Richtung weg vom ersten Zahnkamm (117) allmählich zunimmt, und jede der beiden ersten Führungsflächen (119) als flache Fläche oder als glatt gekrümmte Fläche ausgebildet ist; und
jeder der mehreren zweiten Zähne (156) zwei zweite Führungsflächen (159) aufweist, die symmetrisch zu den beiden Seiten der zweiten Zahnspitze (157) angeordnet sind, und wobei sich der horizontale Abstand zwischen den beiden zweiten Führungsflächen (159) in einer Richtung von der zweiten Zahnspitze (157) weg allmählich vergrößert, und jede der beiden zweiten Führungsflächen (159) ist als flache Fläche oder als sanft gekrümmte Fläche ausgebildet.

4. Die Übertragungs- und Verbindungsstruktur gemäß einem der Ansprüche 1 bis 3, wobei jeder der mehreren ersten Zähne (116) zwei erste Eingriffsflächen (121) aufweist, die symmetrisch auf zwei Seiten eines jeweiligen ersten Zahns (116) angeordnet sind, und jede der beiden ersten Eingriffsflächen (121) als vertikale Fläche oder leicht geneigte Fläche ausgebildet ist;
jeder der mehreren zweiten Zähne (156) zwei zweite Eingriffsflächen (161) aufweist, die symmetrisch auf zwei Seiten eines jeweiligen zweiten Zahns (156) angeordnet sind, und jede der beiden zweiten Eingriffsflächen (161) als vertikale Fläche oder leicht geneigte Fläche ausgebildet ist;
beim Eingriff des ersten Zahnabschnitts (115) mit dem zweiten Zahnabschnitt (155) steht jede der beiden ersten Eingriffsflächen (121) in Kontakt mit einer entsprechenden zweiten Eingriffsfläche (161).

5. Getriebe- und Verbindungsstruktur nach Anspruch 1 oder 3, wobei jeder der mehreren ersten Zähne (116) eine Breite im radialen Querschnitt an der ersten Zahnspitze (117) aufweist, die kleiner ist als eine Breite im radialen Querschnitt an einer Wurzel eines jeweiligen ersten Zahns (116); und/oder
jeder der mehreren zweiten Zähne (156) eine Breite im radialen Querschnitt an der zweiten Zahnspitze (157) aufweist, die kleiner ist als eine Breite im radialen Querschnitt an einer Zahnwurzel eines jeweiligen zweiten Zahns (156).

6. Die Übertragungs- und Verbindungsstruktur gemäß Anspruch 1, wobei der zweite Zahnabschnitt (155) mehrere Verbindungsflächen aufweist, von denen jede zwischen zwei jeweiligen benachbarten zweiten Zähnen (156) des zweiten Zahnabschnitts (155) angeordnet ist, jede der mehreren Verbindungsflächen zwei jeweilige Eingriffsflächen verbindet, die jeweils einem der jeweiligen zwei benachbarten zweiten Zähne (156) entsprechen, und jede der mehreren Verbindungsflächen teilweise als zweite passende Schrägfläche (162) ausgebildet ist, wobei die zweite passende Schrägfläche (162) einen Durchmesser in einem radialen Querschnitt des zweiten Zahnabschnitts (155) aufweist, wobei der Durchmesser der zweiten passenden Schrägfläche (162) entlang einer Richtung von einer zweiten Zahnspitze (157) zu einer Zahnwurzel jedes der mehreren zweiten Zähne (156) allmählich abnimmt, und wobei die zweite passende Schrägfläche (162) so konfiguriert ist, dass sie gleitend mit der ersten glatten Bogenfläche (124) am Umfang des ersten Zahnabschnitts (115) zusammenpasst.

7. Die Übertragungs- und Verbindungsstruktur gemäß Anspruch 6, wobei jeder der ersten Zähne (116) eine erste zylindrische Fläche (125) an einem Umfang des ersten gezahnten Abschnitts (115) aufweist, die erste zylindrische Fläche (125) eine physikalische Fläche des jeweiligen ersten Zahns (116) ist, und die erste zylindrische Fläche (125) an die erste glatte Bogenfläche (124) angrenzt und sich bis zum Fuß jeder der mehreren ersten Zähne (116) erstreckt; und
jede der mehreren Verbindungsflächen teilweise als zweite passende zylindrische Fläche (167) ausgebildet ist, die an die zweite passende geneigte Fläche (162) angrenzt, und die zweite passende zylindrische Fläche (167) so konfiguriert ist, dass sie mit der ersten zylindrischen Fläche (125) des ersten Zahnabschnitts (115) zusammenpasst.

8. Die Übertragungs- und Verbindungsstruktur gemäß Anspruch 1, wobei der Passungsabschnitt so angeordnet ist, dass er dem Positionierungsabschnitt entspricht, und sich der Positionierungsabschnitt zumindest teilweise in den Passungsabschnitt erstreckt.

9. Übertragungs- und Verbindungsstruktur gemäß Anspruch 8, wobei das Übertragungs- und Verbindungselement (151) für den sterilen Adapter (150) ferner ein zweites Antriebsende (152) gegenüber dem zweiten Verbindungsende (153) umfasst.

10. Chirurgischer Roboter (100), umfassend eine Übertragungs- und Verbindungsstruktur gemäß einem der Ansprüche 1 bis 9 und ein chirurgisches Instrument, wobei der sterile Adapter (150) mit dem Instrumentenantrieb (110) verbunden ist und das chirurgische Instrument mit dem sterilen Adapter (150) verbunden ist.

## Revendications

1. Structure de transmission et de connexion, comprenant :
un entraînement d'instrument (110) comprenant un élément de transmission et de connexion (111) pour l'entraînement d'instrument (110), dans lequel l'élément de transmission et de connexion (111) pour l'entraînement d'instrument (110) comporte une première extrémité de connexion (113) configurée pour être couplée à un adaptateur stérile (150), la première extrémité de connexion (113) est pourvue d'une première partie dentée (115), et la première partie dentée (115) comprend une pluralité de premières dents (116) espacées de manière régulière et disposées le long d'une circonférence de l'élément de transmission et de connexion (111) pour l'entraînement d'instrument (110) ; et
l'adaptateur stérile (150) comprenant un élément de transmission et de connexion (151) pour l'adaptateur stérile (150), dans lequel l'élément de transmission et de connexion (151) pour l'adaptateur stérile (150) comporte une deuxième extrémité de connexion (153) configurée pour être couplée à la première extrémité de connexion (113) de l'entraînement d'instrument (110), la deuxième extrémité de connexion (153) est pourvue d'une deuxième partie dentée (155), et la deuxième partie dentée (155) comprend une pluralité de deuxièmes dents (156) espacées de manière régulière et disposées le long d'une circonférence de l'élément de transmission et de connexion (151) pour l'adaptateur stérile (150) ;
pendant le fonctionnement, l'entraînement d'instrument (110) est configuré pour entraîner l'élément de transmission et de connexion (111) pour l'entraînement d'instrument (110) à tourner et à transmettre un couple à un instrument chirurgical via l'élément de transmission et de connexion (151) pour l'adaptateur stérile (150),
dans lequel la deuxième partie dentée (155) est configurée pour s'engrener avec la première partie dentée (115) afin de transmettre le couple via la pluralité de premières dents (116) et la pluralité de deuxièmes dents (156),
chacune de la pluralité de premières dents (116) comporte un premier sommet de dent (117) formé comme un angle vif ou un angle arrondi ; et
chacune de la pluralité de secondes dents (156) comporte une seconde crête de dent (157) formée comme un angle aigu ou un angle arrondi ;
l'élément de transmission et de connexion (111) pour l'entraînement d'instrument (110) comprend en outre une première partie de corps (114) et une partie de positionnement (123) disposée à la première extrémité de connexion (113) et faisant saillie à partir de la première partie de corps (114), la première partie dentée (115) fait saillie à partir d'une surface périphérique extérieure de la partie de positionnement et est disposée autour de la partie de positionnement, la surface périphérique extérieure comprend une première surface inclinée correspondante (122) s'étendant le long d'une circonférence d'une partie supérieure de la partie de positionnement,
dans lequel chacune de la pluralité de premières dents (116) comporte une première surface arquée lisse (124) à la périphérie de la première partie dentée (115), et la première surface arquée lisse (124) a un diamètre dans une section transversale radiale de la première partie dentée (115), le diamètre de la première surface arquée lisse (124) augmentant progressivement le long d'une direction allant d'un premier sommet de dent (117) à une racine de chacune des premières dents respectives (116) ;
dans lequel l'élément de transmission et de connexion (151) pour l'adaptateur stérile (150) comprend en outre une deuxième partie de corps (154) qui est évidée vers l'intérieur au niveau de la deuxième extrémité de connexion (153) pour former une partie d'accouplement (163), la deuxième partie dentée (155) fait saillie à partir d'une surface périphérique interne de la partie d'accouplement et est disposée autour de la partie d'accouplement, la surface périphérique interne comprend une deuxième surface inclinée d'accouplement (162) s'étendant le long d'une circonférence d'une partie inférieure de la partie d'accouplement ;
dans lequel chacune des multiples secondes dents (156) comporte une seconde surface arquée lisse (164) à la périphérie de la seconde partie dentée (155), et la seconde surface arquée lisse (164) a un diamètre dans une section transversale radiale de la seconde partie dentée (155), le diamètre de la deuxième surface arquée lisse (164) diminuant progressivement dans une direction allant d'un deuxième sommet de dent (157) à une racine de chacune des deuxièmes dents respectives (156) ;
dans lequel la première surface arquée lisse (124) est guidée en conjonction avec la deuxième surface inclinée correspondante (162), et la deuxième surface arquée lisse (164) est guidée en conjonction avec la première surface inclinée correspondante (122).

2. Structure de transmission et de connexion selon la revendication 1, dans laquelle chacune de la pluralité de premières dents (116) est symétrique par rapport à une ligne centrale respective, et chacune de la pluralité de secondes dents (156) est symétrique par rapport à une ligne centrale respective.

3. Structure de transmission et de connexion selon la revendication 1, dans laquelle chacune des multiples premières dents (116) comporte deux premières surfaces de guidage (119) disposées symétriquement de part et d'autre du sommet de la première dent (117), respectivement, et la distance horizontale entre les deux premières surfaces de guidage (119) augmente progressivement dans une direction s'éloignant du sommet de la première dent (117), et chacune des deux premières surfaces de guidage (119) est formée comme une surface plane ou une surface courbée en douceur ; et
chacune des multiples secondes dents (156) comporte deux secondes surfaces de guidage (159) disposées symétriquement de part et d'autre du second sommet de dent (157), respectivement, et une distance horizontale entre les deux secondes surfaces de guidage (159) augmente progressivement dans une direction s'éloignant du second sommet de dent (157), et chacune des deux secondes surfaces de guidage (159) est formée comme une surface plane ou une surface légèrement incurvée.

4. Structure de transmission et de connexion selon l'une quelconque des revendications 1 à 3, dans laquelle chacune des multiples premières dents (116) comporte deux premières surfaces d'engagement (121) disposées symétriquement sur deux côtés d'une première dent respective (116), et chacune des deux premières surfaces d'engagement (121) est formée comme une surface verticale ou une surface légèrement inclinée ;
chacune des multiples secondes dents (156) comporte deux secondes surfaces d'engagement (161) disposées symétriquement sur deux côtés d'une seconde dent respective (156), et chacune des deux secondes surfaces d'engagement (161) est formée comme une surface verticale ou une surface légèrement inclinée ;
lors de l'engagement de la première partie dentée (115) avec la deuxième partie dentée (155), chacune des deux premières surfaces d'engagement (121) est en contact avec une deuxième surface d'engagement correspondante (161).

5. Structure de transmission et de connexion selon la revendication 1 ou 3, dans laquelle chacune de la pluralité de premières dents (116) a une largeur dans une section transversale radiale au sommet de la première dent (117) inférieure à une largeur dans une section transversale radiale à la racine d'une première dent respective (116) ; et/ou
chacune des multiples secondes dents (156) présente une largeur dans une section transversale radiale au niveau du second sommet de dent (157) inférieure à une largeur dans une section transversale radiale au niveau d'une racine d'une seconde dent respective (156).

6. Structure de transmission et de connexion selon la revendication 1, dans laquelle la deuxième partie dentée (155) comporte une pluralité de surfaces de connexion, chacune d'entre elles étant disposée entre deux deuxièmes dents adjacentes respectives (156) de la deuxième partie dentée (155), chacune de la pluralité de surfaces de connexion relie deux surfaces d'engagement respectives correspondant chacune à l'une des deux deuxièmes dents adjacentes respectives (156), et chacune de la pluralité de surfaces de connexion est partiellement formée comme la deuxième surface inclinée correspondante (162), la deuxième surface inclinée correspondante (162) a un diamètre dans une section transversale radiale de la deuxième partie dentée (155), le diamètre de la deuxième surface inclinée correspondante (162) diminue progressivement dans une direction allant d'un deuxième sommet de dent (157) à une racine de chacune de la pluralité de deuxièmes dents (156), et la deuxième surface inclinée correspondante (162) est configurée pour s'ajuster de manière coulissante avec la première surface arquée lisse (124) à la périphérie de la première partie dentée (115).

7. Structure de transmission et de connexion selon la revendication 6, dans laquelle chacune des premières dents (116) comporte une première surface cylindrique (125) à la périphérie de la première partie dentée (115), la première surface cylindrique (125) est une surface physique de la première dent respective (116), et la première surface cylindrique (125) est adjacente à la première surface arquée lisse (124) et s'étend jusqu'à la racine de chacune de la pluralité de premières dents (116) ; et
chacune de la pluralité de surfaces de connexion est partiellement formée comme une deuxième surface cylindrique correspondante (167) adjacente à la deuxième surface inclinée correspondante (162), et la deuxième surface cylindrique correspondante (167) est configurée pour s'adapter à la première surface cylindrique (125) de la première partie dentée (115).

8. Structure de transmission et de connexion selon la revendication 1, dans laquelle la partie d'accouplement est agencée pour correspondre à la partie de positionnement, et la partie de positionnement s'étend au moins partiellement dans la partie d'accouplement.

9. Structure de transmission et de connexion selon la revendication 8, dans laquelle l'élément de transmission et de connexion (151) pour l'adaptateur stérile (150) comprend en outre une deuxième extrémité d'entraînement (152) opposée à la deuxième extrémité de connexion (153).

10. Robot chirurgical (100) comprenant une structure de transmission et de connexion selon l'une quelconque des revendications 1 à 9 et un instrument chirurgical, dans lequel l'adaptateur stérile (150) est connecté à l'entraînement d'instrument (110) et l'instrument chirurgical est connecté à l'adaptateur stérile (150).
